# EUROPEAN PATENT APPLICATION

(11) **EP 3 607 921 A1**
(43) Date of publication of application: **12.02.2020**
(21) Application number: 19190124.8
(22) Date of filing: 05.08.2019
(51) Int. Cl.: A61F 5/445, A61F 5/449, A61M 39/02

(54) **CONNECTION DEVICES FOR OSTOMY PROCEDURES**

(30) Priority: 06.08.2018 US 201862715044 P; 22.05.2019 US 201916419037
(71) Applicant: Covidien LP, Mansfield, MA 02048 (US)
(72) Inventor: AVVLN, Srinivasa Murthy Aravalli, 500049 Hyderabad (IN); SURESH, Kumar Prema Mohanasundaram, 602024 Chennai (IN)
(74) Representative: Soames, Candida Jane

(57) **Abstract**

A connection and transfer device for post-operative surgical procedures includes a tubular member having a proximal portion, a distal portion, and a wall defining an inner surface and an outer surface. The distal portion is received within an internal body organ of a patient. The outer surface is configured to interface with an inner wall of the internal body organ. The inner surface of the tubular member defines a through bore dimensioned to allow passage of body fluids. At least one expandable member is disposed on the outer surface of the tubular member and is configured and dimensioned to be received within the internal body organ to engage the inner wall to retain the tubular member within the internal body organ and to prevent the body fluids from passing around the tubular member and into contact with external skin of the patient.

## Description

### CROSS-REFERENCE TO RELATED APPLICATION

This application claims the benefit of and priority to U.S. Provisional Patent Application No. 62/715,044 filed August 6, 2018, the entire disclosure of which is incorporated by reference herein.

### BACKGROUND

### 1. Technical Field

The present disclosure relates to surgical devices and, more particularly, to surgical devices that can be used after ostomy procedures to transfer feces to a ostomy bag without irritating the skin of a patient.

### 2. Description of Related Art

A colostomy is a surgical procedure that brings a portion of the large intestine through the abdominal wall to carry feces out of the body.

A colostomy is a means to treat various disorders of the large intestine, including cancer, obstruction, inflammatory bowel disease, ruptured diverticulum, ischemia (compromised blood supply), or traumatic injury. Temporary colostomies are created to divert stool from injured or diseased portions of the large intestine, allowing rest and healing. Permanent colostomies are performed when the distal bowel must be removed or is blocked and inoperable. Although colorectal cancer is the most common indication for a permanent colostomy, only about 10-15% of patients with this diagnosis require a colostomy.

In current colostomy procedures, the stoma bag or pouch is directly attached to the skin layer. Direct contact between feces and the skin layer causes major post-operative care issues such as infection, skin irritation, leakage, cleaning issues and psychological complications.

### SUMMARY

To address the foregoing disadvantages of the prior art, the present disclosure relates to a connection and transfer device that includes a tubular member having a proximal portion, a distal portion, and a wall defining an inner surface and an outer surface. The distal portion of the tubular member is configured and dimensioned to be received within an internal body organ of a patient. The outer surface of the tubular member is configured to interface with an inner wall of the internal body organ. The inner surface of the tubular member defines a through bore dimensioned to allow passage of body fluids. The device includes at least one expandable member disposed on the outer surface of the tubular member. The at least one expandable member is configured and dimensioned to be received within the internal body organ to engage the inner wall to retain the tubular member within the internal body organ and to prevent the body fluids from passing around the tubular member and into contact with external skin of the patient.

In an aspect, the device includes a threaded cap, wherein the proximal and distal portions of the tubular member define proximal and distal open ends, the proximal portion including a threaded surface extending along the outer surface of the wall, and the threaded surface is configured to engage the threaded cap to secure the threaded cap on the tubular member to close the proximal open end.

In an aspect, the tubular member includes a serrated surface having elevated ridges that project from at least a portion of the outer surface. The elevated ridges are configured to engage the inner wall of the internal body organ to retain the proximal portion of the tubular member within the internal body organ.

In an aspect, the device includes an inflation channel including an inlet port and an outlet port, the inflation channel extending peripherally from the outer surface of the tubular member and continuing distally along the wall of the tubular member to interface with the at least one expandable member, wherein the at least one expandable member includes an inflatable member.

In an aspect, the device includes a partial ring structural member configured to extend around the proximal portion of the outer surface of the wall. The partial ring structural member is configured to engage the external skin of the patient to prevent the proximal portion of the tubular member from passing into a body cavity of the patient.

In an aspect, the at least one expandable member is configured and disposed around the elevated ridges to seal the interior side of the internal body organ when the at least one inflatable member is inflated.

In another aspect, the tubular member includes a first tubular member and a second tubular member that are movable in relation to each other. Each of the first and second tubular members includes a proximal portion and a distal portion and defines an inner surface and an outer surface. The first tubular member defines a centerline axis. The second tubular member is configured to extend around the outer surface of the first tubular member. The at least one expandable member includes a first set of movable links and a second set of movable links. At least two of the first set of movable links are operably coupled to a proximal portion of the first tubular member and to at least two of the second set of movable links that are operably coupled to a distal portion of the second tubular member such that, upon movement proximally of the first tubular member along the inner surface of the second tubular member, the first and second set of movable links move outwardly with respect to the centerline axis to engage the inner wall to retain the first and second tubular members within the internal body organ and to prevent the body fluids from passing around the first and second tubular members and into contact with external skin of the patient.

In an aspect, the device further includes a retaining ring structural member, wherein the outer surface of the first tubular member defines a circumferential groove extending around the centerline axis. The circumferential groove is disposed such that when the first tubular member is inserted internally through the internal body organ, the circumferential groove is positioned externally of a body cavity of the patient to receive the retaining ring structural member, the retaining ring structural member being positioned and configured to inhibit movement of the proximal portion of the connection and transfer device into the body cavity.

In an aspect, the device includes a flexible collection receptacle member disposed on the proximal portion of the first tubular member.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure:
FIG. 1 is an exploded view of an aspect of a connection and transfer device according to an exemplary embodiment of the present disclosure that includes a serrated sleeve and related components that enable transfer of feces directly to an ostomy bag or pouch without contacting the skin of a patient;
FIG. 2 is a cross-sectional view of the connection and transfer device of FIG. 1 taken along cross-section line 2-2 in FIG. 1 illustrating the connection and transfer device penetrating the abdominal wall of a patient with a sealing balloon in an uninflated position;
FIG. 3 is a cross-sectional view of the connection and transfer device of FIG. 2 with the sealing balloon in an inflated position to seal the serrated sleeve with respect to the abdominal wall of the patient;
FIG. 4 is a perspective view of another exemplary embodiment of a connection and transfer device according to the present disclosure that includes hinged connector links positioned around a sleeve that enables transfer of feces directly to an ostomy bag or pouch without contacting the skin of a patient;
FIG. 5 is an exploded perspective view of the connection and transfer device of FIG. 4;
FIG. 6 is a cross-sectional view of the connection and transfer device taken along section line 6-6 of FIG. 4 illustrating the connection and transfer device penetrating the abdominal wall of a patient with the hinged connector links in a straight and un-extended position;
FIG. 7 is a perspective view of the connection and transfer device of FIGS. 4 with the hinged connector links in an extended position; and
FIG. 8 is a cross-sectional view taken along section line 6-6 of FIG. 4 with the hinged connector links of the connection and transfer device in an extended position to seal the sleeve with respect to the abdominal wall of the patient.

### DETAILED DESCRIPTION

Aspects of the present disclosure relate to connection and transfer devices that provide significant and non-obvious advantages in the field of ostomy bags or pouches by enabling transfer of feces through a stoma without the feces contacting the skin of a patient.

Throughout this description, the term "proximal" refers to the portion of the device closest to the operator and the term "distal" refers to the portion of the device furthest from the operator.

The present disclosure addresses issues that exist in current colostomy procedures wherein the stoma bag/pouch is directly attached to a skin layer so as to cause major post-operative care issues such as infection, skin irritation, leakage, cleaning issues and psychological complications when feces comes in direct contact with the skin layer of a patient. The present disclosure relates to significant and non-obvious devices that yield major non-obvious advantages by preventing contact of feces with the skin layer, thereby avoiding the foregoing major post-operative care issues.

More particularly, the present disclosure relates to connector devices each of which acts as a bridge to transfer the feces directly into the ostomy bag or pouch without contacting the skin layer.

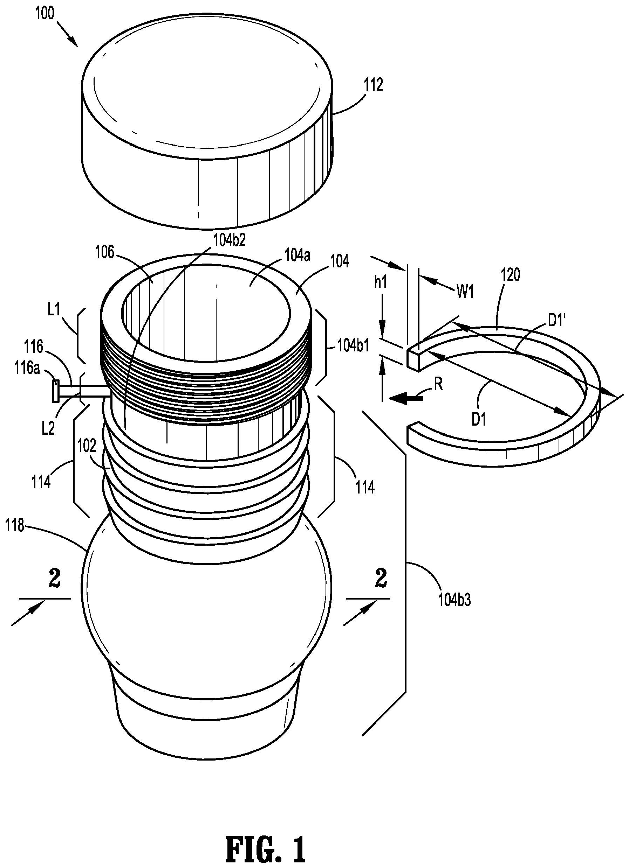

Referring to FIGS. 1-3, a connection and transfer device 100 for post-operative surgical procedures includes a tubular member 102 including a wall 104 defining an inner surface 104a and an outer surface 104b. The tubular member 102 defines a proximal open end portion 106 and a distal open end portion 108. The outer surface 104b includes a first portion 104b1, a second portion 104b2 extending from the first portion 104b1 and a third portion 104b3 extending from the second portion 104b1.

The first portion 104blof the outer surface 104b includes a male engaging surface 110 extending from the proximal end portion 106 to define a first length L1 along the outer surface 104b of the wall 104. The male engaging surface 110 is configured to engage with a female engaging cap 112. In aspects of the present disclosure, the male engaging surface 110 and the female engaging cap 112 include intermeshing threaded surfaces, although other engaging structures may be utilized such as, for example, bayonet type couplings, snap-on caps or the like. The cap 112 seals the end of the tubular member 102 to prevent feces from exiting the connection and transfer device 100.

The second portion 104b2 of the outer surface 104b of the wall 104 extends distally from termination of the male engaging surface 110 to define a second length **L2** extending to the beginning of a serrated surface of elevated ridges 114 projecting from, and part of, the outer surface 104b, towards the distal open end portion 108. The serrated surface of elevated ridges 114 define a third length **L3** along the outer surface 104b of the wall 104, corresponding to the third portion of the outer surface 104b3. The tubular member 102 is configured for insertion internally through a stoma **"S"** that extends from the exterior side 20' of a patient incision 22 through the skin 20 and the through the interior side 20" of the patient incision 22 and into an internal organ **"I"** such as the large intestine or the small intestine. An inlet port 116a of an inflation channel 116 extends peripherally from the second portion 104b2 of the outer surface 104b and continues distally within the wall 104 for a distance **D** to extend peripherally to define an outlet port 116b of the inflation channel 116. The outlet port 116b is configured to interface with an inflatable circumferential member 118 that is configured and disposed to seal the interior side 20" of the patient incision 22 when the inflatable member 118 is in an inflated, pressurized configuration. The distance **D** is sufficient to enable the inflation channel 116 to extend distally from the exterior side 20' of the patient incision 22 and distally from the interior side 20" and interface with the inflatable circumferential member 118 to enable the inflation and pressurization of the member 118.

A partial ring structural member 120is configured to extend around the second portion 104b2 of the outer surface 104b of the wall 104 to secure the connection and transfer device 100 on the exterior side 20' of the patient incision 22. More specifically, the outer diameter **"D1"'** of the structural member 120, defined by the sum of the internal diameter **"D1"** plus twice the width thickness **"w1",** is selected to prevent the connection and transfer device 100 from passing into the incision 22. The internal diameter **"D1"** is selected to generally match the outer diameter defined by the second portion 104b2 of the outer surface 104b of the wall 104 to enable a secure fit or engagement of the partial ring structural member 120 to the wall 104.

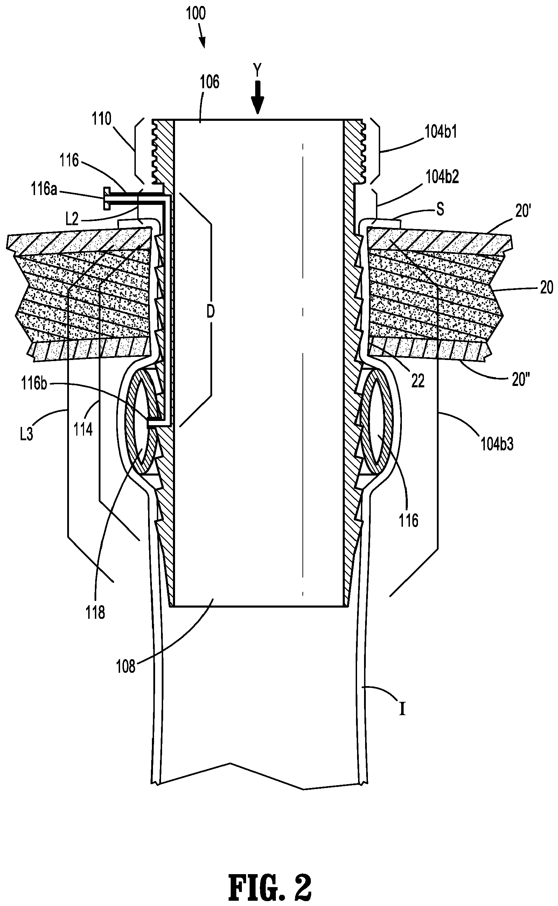

Referring to FIG. 2, a method of applying the connector and transfer device 100 according to an aspect of the present disclosure includes inserting the tubular member 102 configured and dimensioned with the serrated surface of elevated ridges 114 into the stoma **"S"** in the direction of arrow **Y.**

An ostomy bag or pouch (not shown) will be connected to the outer surface 104b of the wall 104 at the proximal first portion 104b1 that includes male engaging surface 110.

The step of inserting the tubular member 102 into the stoma **"S"** includes retaining the tubular member 102 within the stoma **"S"** with the inflatable circumferential member 118 and with the serrated surface of elevated ridges 114 in the abdominal cavity.

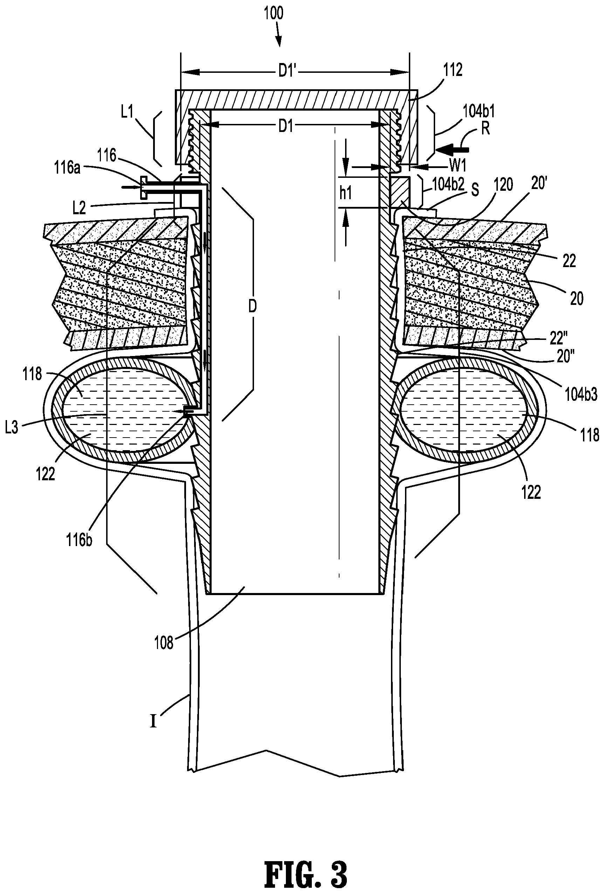

Referring to FIG. 3, in some embodiments, the method includes injecting liquid 122, e.g., water or saline solution, via the inlet port 116a into the inflatable circumferential member 118 and sealing the inlet port 116a with a unidirectional valve (not shown) such that the inflatable circumferential member 118 expands and grips against the serrated surface of elevated ridges 114 to secure the connector and transfer device 100 within the stoma **"S".**

The method may also include installing the partial ring structural member 120 in the direction of arrow **R** to extend around the second portion 104b2 of the outer surface 104b of the wall 104 of the tubular member 102 to secure the connection and transfer device 100 on the exterior side 20' of the patient incision 22. The outer diameter **"D1'"** of the partial ring structural member 120 is made to be sufficient to inhibit the tubular member 102 from passing into the incision 22.

In addition to width thickness **"w1",** height thickness **"h1"** of the structural member 120 also serves to inhibit movement of the connection and transfer device 100 into the incision 22.

In some embodiments, the method includes installing the female engaging member 112 on the male engaging surface 110 or installing an ostomy pouch or bag (not shown) on the proximal open end portion 106 of the tubular member 102.

Thus, those skilled in the art will understand that the connector and transfer device 100 is intended to be inserted into a stoma (either a temporary or permanent ileostomy or colostomy) and includes a cap or female engaging member on its exterior portion that will enable the user or patient or caregiver to avoid an ostomy pouch or bag for certain periods of time as desired.

The device 100 is anchored or fixed within the stoma **"S"** and an internal organ **"I"** using the elevated ridges 114 on the tubular member 102 and the inflatable circumferential member 118. The inflatable circumferential member 118 expands when injected with liquid or water 122 thru the external orifice 116a by the user.

The expanded inflatable circumferential member 118 engages the serrations of the tubular member 102 and holds the device 100 in place within the stoma **"S"** and the internal body organ **"I"**.

That is, the expanded inflatable circumferential member 118 is configured and dimensioned to be received within the internal body organ **"I"** to engage the inner wall or peritoneum wall and holds it against the peritoneum wall (the interior side 20" of the patient skin 20 from the incision 22) to retain the tubular member 102 within the internal body organ **"I"** and to prevent the body fluids from passing around the tubular member 102 and into contact with external skin of the patient.

The inflatable circumferential member 118 may be made from biocompatible flexible material to avoid injury to an internal surface of the body organ **"I".**

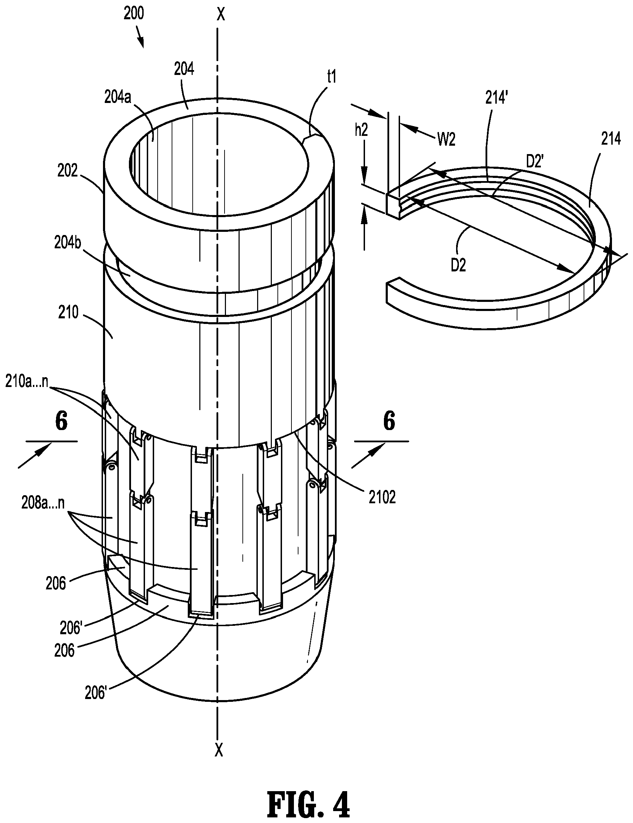

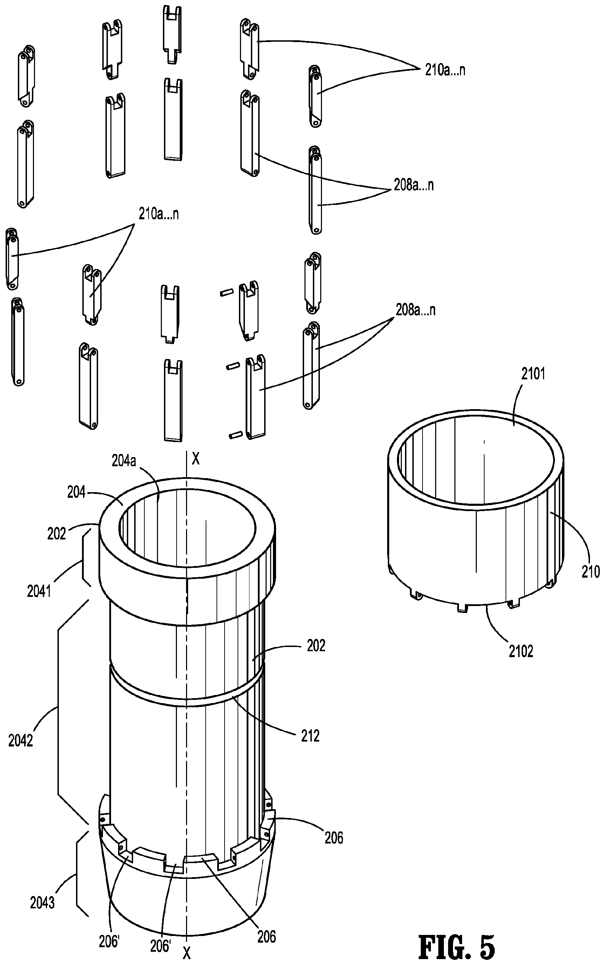

FIGS. 4-8 illustrate another exemplary embodiment of the presently disclosed connection and transfer device shown generally as 200. The connection and transfer device 200 includes a first tubular member 202 having a wall 204 defining an inner surface 204a and an outer surface 204b. The first tubular member 202 defines a centerline axis **X-X.**

The tubular member 202 and wall 204 include a first portion 2041, a second portion 2042 extending from the first portion 2041 and a third portion 2043 extending from the second portion 2042.

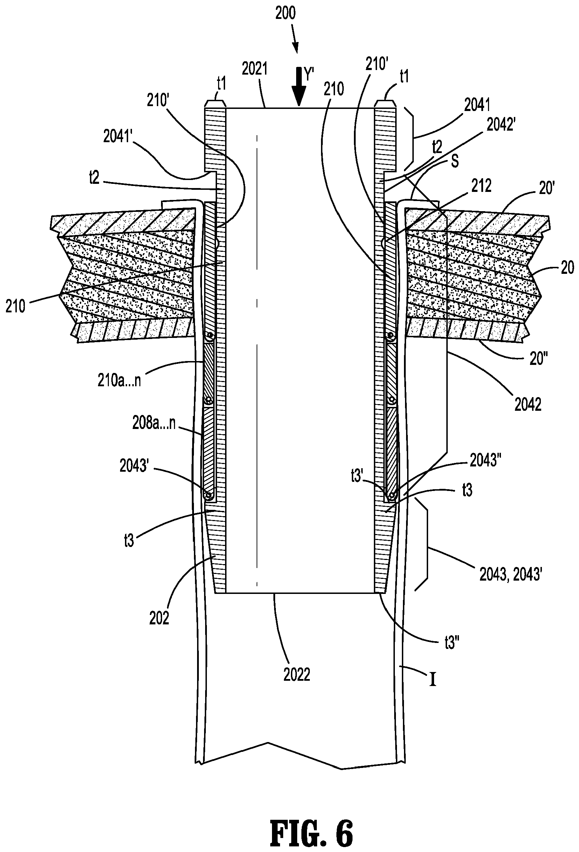

Referring to FIG. 6, the first portion of the wall 2041 has a wall thickness **t1.** Similarly, the second portion of the wall 2042 has a wall thickness **t2.** The third portion of the wall 2043 has a wall thickness **t3** that varies distally from a maximum **t3'** at the intersection with the second portion of the wall 2042 to a minimum **t3"** at the distal open end portion 2022.

The first tubular member 202 defines a proximal open end portion 2021 of the first portion 2041 and a distal open end portion 2022 of the third portion 2043,

The proximal open end portion 2021 of the first portion of the wall 2041 includes a concentric outwardly extending portion 2041' extending along the first portion 2041 with respect to the centerline axis **X-X** such that the wall thickness **t1** of the first portion 2041 is greater than the wall thickness **t2** of the second portion 2042.

The second portion 2042 extends to the third portion 2043. The distal open end portion 2022 of the third portion 2043 includes a concentric outwardly extending sloped portion 2043' extending along the third portion 2043 with respect to the centerline axis **X-X** such that the wall thickness **t3** of the third portion 2043 is greater than the wall thickness **t2** of the second portion.

The third portion 2043 includes a proximal end portion 2043" that defines intermittent projections 206 further extending proximally and concentrically around the centerline axis **X-X** to further define a series of gaps 206' between the intermittent projections 206.

The gaps 206' between the intermittent projections 206 are each configured to receive a first set of a plurality of movable links 208a...n. The set of a plurality of movable links 208a...n are operably coupled to the proximal end portion 2043" of the third portion 2043. At least two, if not each, of the plurality of movable links 208a...n are configured to interconnect with at least two, if not each, of a second set of a plurality of movable links 210a...n. The moveable links 210a...n extend from a distal end portion 2102 of a second tubular member 210. The second tubular member 210, having a proximal end portion 2101, is configured to extend around the outer surface 2042' of the second portion 2042 of the first tubular member 202. Therefore, the second tubular member 210 includes an inner surface 210' that interfaces with the outer surface 2042' of the first tubular member 202.

As with respect to connection and transfer device 100, the first tubular member 202 is configured and dimensioned for insertion into the stoma **"S"** and into a portion of an internal body organ **"I",** e.g., small or large intestine, that extends from the exterior side 20' of patient incision 22 through the skin 20 and the through the interior side 20" of the patient incision 22.

The first set of a plurality of movable links 208a...n are received in the gaps 206' between the intermittent projections 206 and are operably coupled with the proximal end portion 2043" of the third portion 2043.

The second set of a plurality of movable links 210a...n are operably coupled with the distal end portion 2102 of second tubular member 210 and made from a bio-compatible flexible material. The links 210a...n are configured such that upon movement proximally of the first tubular member 202 in the direction of the centerline axis **X-X** along the inner surface 210' of the second tubular member 210, as indicated by arrow **A,** at least two, if not each, of the plurality of movable links 210a...n move outwardly with respect to the centerline axis **X-X** in the direction of arrows **B.** Outward movement of the links 210a...n effects joint outward movement of the plurality of movable links 208a...n that are operably coupled with the proximal end portion 2043" of the third portion 2043 of the first tubular member 202.

The plurality of movable links 210a...n operably coupled with the distal end portion 2102 of second tubular member 210 extend along the interior side 20" of the patient incision 22 and into the internal organ **"I"** when the first tubular member 202 is inserted through stoma **"S".**

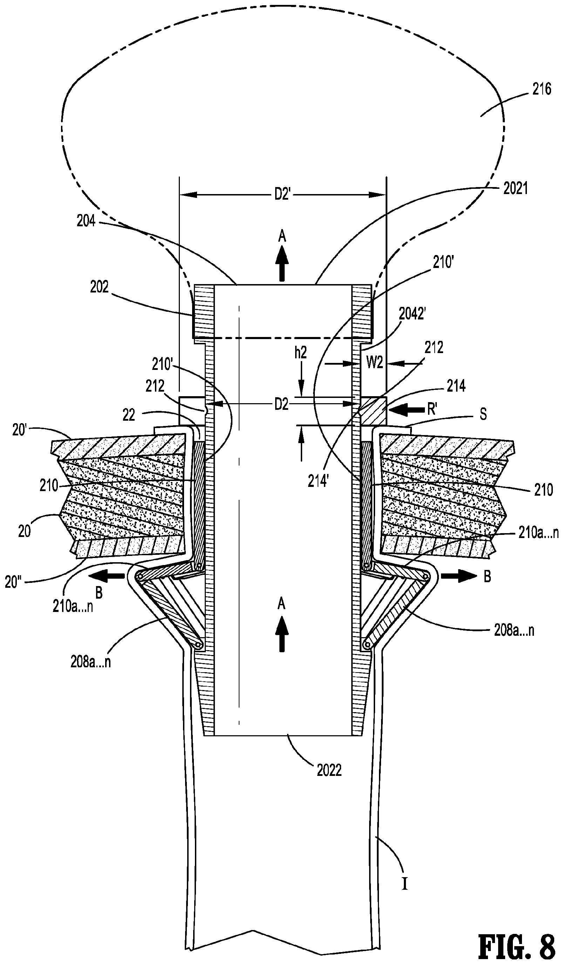

Referring to FIG. 8, the connection and transfer device 200 may further include a circumferential groove 212 that is defined in the outer surface 2042' of the second portion 2042 of the first tubular member 202 and extends around the centerline axis **X-X.** The circumferential groove 212 is disposed such that when the first tubular member 202 is inserted through stoma **"S",** the circumferential groove 212 is positioned on the exterior side 20' of the patient incision 22 to enable receipt of a retaining ring structural member 214 therein to inhibit movement of the connection and transfer device 200 further into the stoma **"S".**

In a similar manner as with respect to partial ring structural member 120 described above, the outer diameter **"D2"'** of the retaining ring structural member 214, defined by the sum of the internal diameter **"D2"** plus twice the width thickness **"w2",** is selected to inhibit the first portion of the wall 2041 of the wall 204 of first tubular member 202 from passing into the incision 22. More specifically, the interior surface of the retaining ring structural member 214 includes a raised protrusion 214' that is received in the circumferential groove 212 positioned on the exterior side 20' of the patient incision 22. The internal diameter **"D2"** of retaining ring structural member 214 is dimensioned to enable engaging the outer surface 2042' and so the retaining ring structural member 214 and its raised protrusion 214' inhibit movement of the connection and transfer device 200 into the incision 22.

In addition to width thickness **"w2",** also as with respect to partial ring structural member 120 described above, height thickness **"h2"** of the retaining ring structural member 214 also serves to inhibit movement of the connection and transfer device 200 into the incision 22.

In some embodiments, the connection and transfer device 200 further includes a flexible collection receptacle member 216 that is disposed on the proximal open end portion 2021 of the first portion 2041 of the first tubular member 202 to enable transfer of feces through the stoma **"S"** and collection of the feces within the flexible collection receptacle member 216 without the feces contacting the skin of a patient.

Referring to FIG. 6, a method of applying the connector and transfer device 200 according to an aspect of the present disclosure includes inserting the distal open end portion 2022 of the device 200, with the links 208a...n and 210a...n in co-linear positions flush against the second portion 2042 of the first tubular member 202, through the stoma **"S"** and into the internal organ **"I"** in the direction of arrow **Y'** with flexible collection receptacle member 216, e.g., an ostomy pouch or bag, connected to the proximal open end portion 2021 of the first portion 2041.

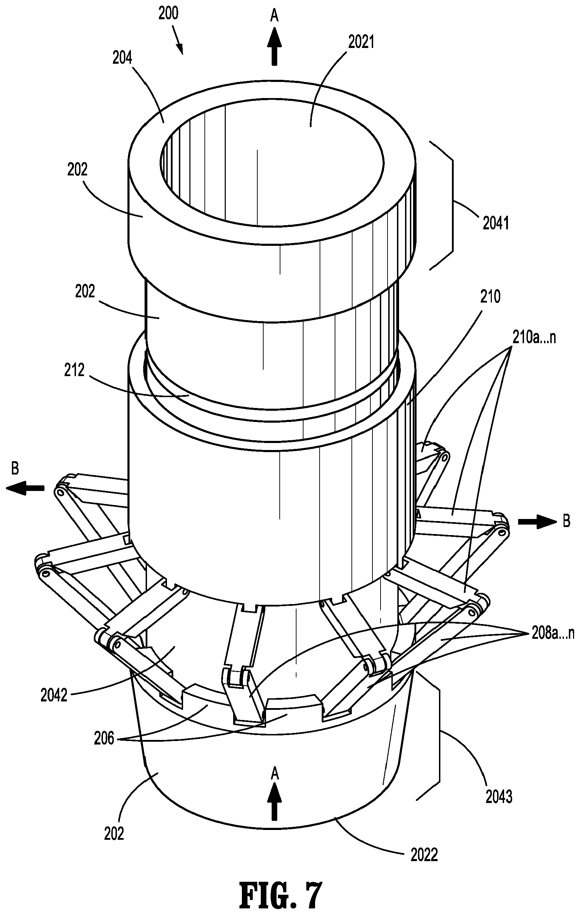

The method further includes a user or patient or caregiver pulling, either with or without an instrument, the first tubular member 202 proximally in the direction of arrow **A** outwards as shown in FIGS. 7-8 along the inner surface 210' of the second tubular member 210 to move the first tubular member 202 in relation to the second tubular member 210. This relative movement causes the links 208a...n and 210a...n to transfer or pivot from the co-linear positions to extended positions in the direction of arrows **B** such that the movable links 210a...n extending from the distal end portion 2102 of the second tubular member 210 now extend generally along the interior side 20" of the patient skin 20 from the incision 22.The transferring or pivoting of the links 208a...n and 210a...n from the co-linear positions to extended positions expands and enlarges the internal body organ **"I"** uniformly and holds it against the peritoneum wall (the interior side 20" of the patient skin 20 from the incision 22).

That is, at least two movable links 210a...n of the second set of movable links extend to be received within the internal body organ **"I"** to engage the inner wall to retain the first and second tubular members 202 and 210, respectively, within the internal body organ **"I"** and to prevent the body fluids from passing around the tubular members 202 and 210 and into contact with external skin of the patient.

In embodiments, the method generally includes inserting retaining ring structural member 214 in the circumferential groove 212 in the first tubular 202 that is positioned on the exterior side 20' of the patient incision 22 in the direction of arrow **R',** thereby securing the connection and transfer device 200 in the stoma **"S"** in a locked position.

The connection and transfer device 200 thereby reduces post-operative care procedures and the possibility of infection as there is no direct contact of fecal material with ostomy sutures or the patient skin.

Additionally, the possibility of allergic reactions is reduced since contact of the fecal material and the pouch with the skin is reduced or eliminated. Leakage and cleaning issues are reduced as well as psychological complications, thereby improving patient life style.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as examples of particular embodiments. Those skilled in the art will envision other modifications within the scope and spirit of the claims appended hereto.

Although the foregoing disclosure has been described in some detail by way of illustration and example, for purposes of clarity or understanding, it will be obvious that certain changes and modifications may be practiced within the scope of the appended claims.

The invention may be described by reference to the following numbered paragraphs:-
1. A connection and transfer device comprising:
   a tubular member having a proximal portion, a distal portion, and a wall defining an inner surface and an outer surface, the distal portion of the tubular member configured and dimensioned to be received within an internal body organ of a patient, the outer surface of the tubular member configured to interface with an inner wall of the internal body organ, the inner surface of the tubular member defining a through bore dimensioned to allow passage of body fluids; and
   at least one expandable member disposed on the outer surface of the tubular member, the at least one expandable member being configured and dimensioned to be received within the internal body organ to engage the inner wall to retain the tubular member within the internal body organ and to prevent the body fluids from passing around the tubular member and into contact with external skin of the patient.
2. The connection and transfer device according to paragraph 1, further including a threaded cap, wherein the proximal and distal portions of the tubular member defining proximal and distal open ends, the proximal portion including a threaded surface extending along the outer surface of the wall, the threaded surface configured to engage the threaded cap to secure the threaded cap on the tubular member to close the proximal open end.
3. The connection and transfer device according to paragraph 2, wherein the tubular member includes a serrated surface having elevated ridges that project from at least a portion of the outer surface, the elevated ridges being configured to engage the inner wall of the internal body organ to retain the proximal portion of the tubular member within the internal body organ.
4. The connection and transfer device according to paragraph 3, further including
   an inflation channel including an inlet port and an outlet port, the inflation channel extending peripherally from the outer surface of the tubular member and continuing distally along the wall of the tubular member to interface with the at least one expandable member, wherein the at least one expandable member includes an inflatable member.
5. The connection and transfer device according to paragraph 2, further including a partial ring structural member configured to extend around the proximal portion of the outer surface of the wall, the partial ring structural member being configured to engage the external skin of the patient to prevent the proximal portion of the tubular member from passing into a body cavity of the patient.
6. The connection and transfer device according to paragraph 4, wherein the at least one expandable member is configured and disposed around the elevated ridges to seal the interior side of the internal body organ when the at least one inflatable member is inflated.
7. The connection and transfer device according to paragraph 1, wherein the tubular member includes:
   a first tubular member and a second tubular member that are movable in relation to each other,
      each of the first and second tubular members including a proximal portion and a distal portion and defining an inner surface and an outer surface,
      the first tubular member defining a centerline axis,
      the second tubular member configured to extend around the outer surface of the first tubular member,
   wherein the at least one expandable member includes a first set of movable links and a second set of movable links, at least two of the first set of movable links operably coupled to a proximal portion of the first tubular member and to at least two of the second set of movable links that are operably coupled to a distal portion of the second tubular member such that, upon movement proximally of the first tubular member along the inner surface of the second tubular member, the first and second set of movable links move outwardly with respect to the centerline axis to engage the inner wall to retain the first and second tubular members within the internal body organ and to prevent the body fluids from passing around the first and second tubular members and into contact with external skin of the patient.
8. The connection and transfer device according to paragraph 7, further including:
   a retaining ring structural member,
   wherein the outer surface of the first tubular member defines a circumferential groove extending around the centerline axis, the circumferential groove disposed such that when the first tubular member is inserted internally through the internal body organ, the circumferential groove is positioned externally of a body cavity of the patient to receive the retaining ring structural member, the retaining ring structural member being positioned and configured to inhibit movement of the proximal portion of the connection and transfer device into the body cavity.
9. The connection and transfer device according to paragraph 7, further including a flexible collection receptacle member disposed on the proximal portion of the first tubular member.

## Claims

1. A connection and transfer device comprising:
a tubular member having a proximal portion, a distal portion, and a wall defining an inner surface and an outer surface, the distal portion of the tubular member configured and dimensioned to be received within an internal body organ of a patient, the outer surface of the tubular member configured to interface with an inner wall of the internal body organ, the inner surface of the tubular member defining a through bore dimensioned to allow passage of body fluids; and
at least one expandable member disposed on the outer surface of the tubular member, the at least one expandable member being configured and dimensioned to be received within the internal body organ to engage the inner wall to retain the tubular member within the internal body organ and to prevent the body fluids from passing around the tubular member and into contact with external skin of the patient.

2. The connection and transfer device according to claim 1, further including a threaded cap, wherein the proximal and distal portions of the tubular member defining proximal and distal open ends, the proximal portion including a threaded surface extending along the outer surface of the wall, the threaded surface configured to engage the threaded cap to secure the threaded cap on the tubular member to close the proximal open end.

3. The connection and transfer device according to claim 2, wherein the tubular member includes a serrated surface having elevated ridges that project from at least a portion of the outer surface, the elevated ridges being configured to engage the inner wall of the internal body organ to retain the proximal portion of the tubular member within the internal body organ.

4. The connection and transfer device according to claim 3, further including
an inflation channel including an inlet port and an outlet port, the inflation channel extending peripherally from the outer surface of the tubular member and continuing distally along the wall of the tubular member to interface with the at least one expandable member, wherein the at least one expandable member includes an inflatable member.

5. The connection and transfer device according to claim 2, further including a partial ring structural member configured to extend around the proximal portion of the outer surface of the wall, the partial ring structural member being configured to engage the external skin of the patient to prevent the proximal portion of the tubular member from passing into a body cavity of the patient.

6. The connection and transfer device according to claim 4, wherein the at least one expandable member is configured and disposed around the elevated ridges to seal the interior side of the internal body organ when the at least one inflatable member is inflated.

7. The connection and transfer device according to any preceding claim, wherein the tubular member includes:
a first tubular member and a second tubular member that are movable in relation to each other,
each of the first and second tubular members including a proximal portion and a distal portion and defining an inner surface and an outer surface,
the first tubular member defining a centerline axis,
the second tubular member configured to extend around the outer surface of the first tubular member,
wherein the at least one expandable member includes a first set of movable links and a second set of movable links, at least two of the first set of movable links operably coupled to a proximal portion of the first tubular member and to at least two of the second set of movable links that are operably coupled to a distal portion of the second tubular member such that, upon movement proximally of the first tubular member along the inner surface of the second tubular member, the first and second set of movable links move outwardly with respect to the centerline axis to engage the inner wall to retain the first and second tubular members within the internal body organ and to prevent the body fluids from passing around the first and second tubular members and into contact with external skin of the patient.

8. The connection and transfer device according to claim 7, further including:
a retaining ring structural member,
wherein the outer surface of the first tubular member defines a circumferential groove extending around the centerline axis, the circumferential groove disposed such that when the first tubular member is inserted internally through the internal body organ, the circumferential groove is positioned externally of a body cavity of the patient to receive the retaining ring structural member, the retaining ring structural member being positioned and configured to inhibit movement of the proximal portion of the connection and transfer device into the body cavity.

9. The connection and transfer device according to claim 7, further including a flexible collection receptacle member disposed on the proximal portion of the first tubular member.
